Europäisches Patentamt

European Patent Office

Office européen des brevets

(11) Publication number: **0 095 452**
**A2**

# (12) EUROPEAN PATENT APPLICATION

(21) Application number: **83870048.2**

(22) Date of filing: **18.05.83**

(51) Int. Cl.³: **C 12 N 15/00**
**C 12 P 21/02, C 07 C 103/52**
**C 07 H 21/04, A 61 K 45/02**
**C 12 N 1/20**
**//C12R1/19, C12R1/63**

(30) Priority: **24.05.82 US 381083**

(43) Date of publication of application:
**30.11.83 Bulletin 83/48**

(84) Designated Contracting States:
**AT BE CH DE FR GB IT LI LU NL SE**

(71) Applicant: **Smith Kline - RIT Société anonyme dite:**
**rue du Tilleul, 13**
**B-1320 Rixensart Genval(BE)**

(72) Inventor: **Harford, Nigel**
**Jef Lambeaulaan 9**
**B-1900 Overijse(BE)**

(72) Inventor: **De Wilde, Michel**
**Avenue Gevaert 136**
**B-1320 Genval(BE)**

(74) Representative: **Tasset, Gérard**
**Smith Kline - RIT rue de l'Institut, 89**
**B-1330 Rixensart(BE)**

(54) **Preparation of DNA sequences and DNA recombinant molecules coding for the cholera toxine units A and B, the products obtained and compositions containing the unit(s) obtained.**

(57) Des séquences d'ADN et des molécules d'ADN recombinantes comprenant au moins une portion codant pour la totalité ou pour une partie des sous-unités A et/ou B de la toxine cholérique sont préparées par digestion enzymatique de l'ADN de souches de *V. cholerae,* en isolant les fragments spécifiques et en les insérant dans des vecteurs appropriés et les sous-unités A et/ou B de la toxine cholérique sont préparées par culture des microorganismes contenant les vecteurs modifiés.

EP 0 095 452 A2

PREPARATION DE SEQUENCES D'ADN ET MOLECULES D'ADN RECOMBINANTES CODANT POUR LES SOUS-UNITES A ET B DE LA TOXINE CHOLERIQUE, PRODUITS OBTENUS ET PREPARATIONS CONTENANT LA OU LES SOUS-UNITE(S) OBTENUE(S).

La présente invention concerne la préparation de séquences d'ADN et molécules d'ADN recombinantes codant pour les sous-unités A et B de la toxine cholérique, produits obtenus et préparations contenant la ou les sous-unité(s) obtenue(s).

On sait que l'holotoxine produite par certaines souches de Vibrio cholerae se compose de deux sous-unités protéiniques qui sont connues sous le nom de sous-unités A et B.

La sous-unité A (TCA) est responsable de la pénétration de la cellule épithéliale et de l'effet enzymatique qui conduit à une importante perte de liquide dans l'intestin tandis que la sous-unité B (TCB) fixe l'holotoxine aux sites récepteurs du monosialosylganglioside de type $G_{M1}$ sur la paroi cellulaire; elle ne possède aucune activité toxique et est fortement immunogène.

Parmi les souches de Vibrio cholerae produisant l'holotoxine, on peut citer, par exemple, les souches de Vibrio cholerae biotype El Tor sérotype INABA et, dans Proc. Natl. Acad. Sci. EUA 76, 2052-2056, 1979, T. HONDA et R.A. FINKELSTEIN décrivent un mutant de Vibrio cholerae qui produit la sous unité B de la toxine cholérique mais aucune quantité détectable de la sous-unité A.

Les vaccins cholériques actuels contenant soit des lipopolysaccharides extraits de vibrions, soit des suspensions denses de vibrions tués peuvent, après injections répétées, conférer une protection limitée aux sujets en contact et fortement exposés mais ils s'avèrent inefficaces en tant que moyen de suppression de l'épidémie. Chez des volontaires humains, après guérison du choléra, on a également démontré la présence d'anticorps à l'égard de l'organisme homologue ainsi qu'un degré élevé de résistance à l'égard de celui-ci, mais pas à l'égard des souches hétérologues.

Considérant que l'holotoxine cholérique est fortement antigénique et qu'il n'existe seulement qu'une variété immunogène, on a suggéré qu'une immunité antitoxique efficace pourrait protéger contre les différents sérotypes et biotypes de V. cholerae.

A cette fin, la sous-unité B semble être un composant vaccinal souhaitable pour induire des anticorps protecteurs soit par administration orale ou parentérale et différents auteurs ont proposé des vaccins anticholériques basés sur la toxine de la sous-unité B ou, avec une préférence moindre, sur les antitoxines (J. HOLMGREN et al. dans Nature 269, 602-603, 1977; HONDA et R.A. FINKELSTEIN dans loc. cit. et J. HOLMGREN dans Nature 292, 413-417, 1981).

Avant la présente invention, on savait également que l'holotoxine est déterminée par un gène chromosomique (ctx) et S.L. MOSELEY et S. FALKOW (J. Bact. 144, 444-446, 1980) ont montré que les fragments d'ADN représentant les deux cistrons du gène apparenté à l'entérotoxine thermolabile (elt) de E. coli peuvent être utilisés comme sondes au cours des expériences d'hybridation ADN/ADN pour détecter les fragments spécifiques du gène ctx composant l'ADN total de V. cholerae digéré par les différentes endonucléases de restriction.

La demanderesse a découvert et ceci fait l'objet de la présente invention que la digestion de l'ADN d'une souche toxigénique de Vibrio cholerae produisant l'holotoxine (laquelle souche est exemplifiée ici par une souche de Vibrio cholerae El Tor INABA qui a été déposée à l'American Type Culture Collection, Rockville, Maryland, E.U.A. sous le numéro d'ordre ATCC 39050) par l'endonucléase ClaI libère deux bandes d'ADN distinctes qui s'hybrident respectivement et exclusivement aux sondes eltB et eltA et renferment par conséquent les cistrons ctxB et ctxA apparentés.

Suivant l'invention, les fragments contenant soit le gène ctxA, soit le gène ctxB et obtenus par traitement à l'aide de l'endonucléase ClaI, comme indiqué ci-dessus, sont insérés soit séparément soit séquentiellement dans un vecteur approprié qui a été préalablement clivé par l'endonucléase ClaI pour constituer des vecteurs de clonage dans des microorganismes hôtes tels que, par exemple, une souche d'E. coli K12 ou une souche non toxigénique de V. cholerae. Les souches finalement obtenues sont utilisées ensuite pour la production de la sous-unité A ou de la sous-unité B ou des deux sous-unités A et B de la toxine cholérique ou des dérivés génétiquement modifiés de celle-ci.

Plus particulièrement et ainsi qu'il est exemplifié ci-après

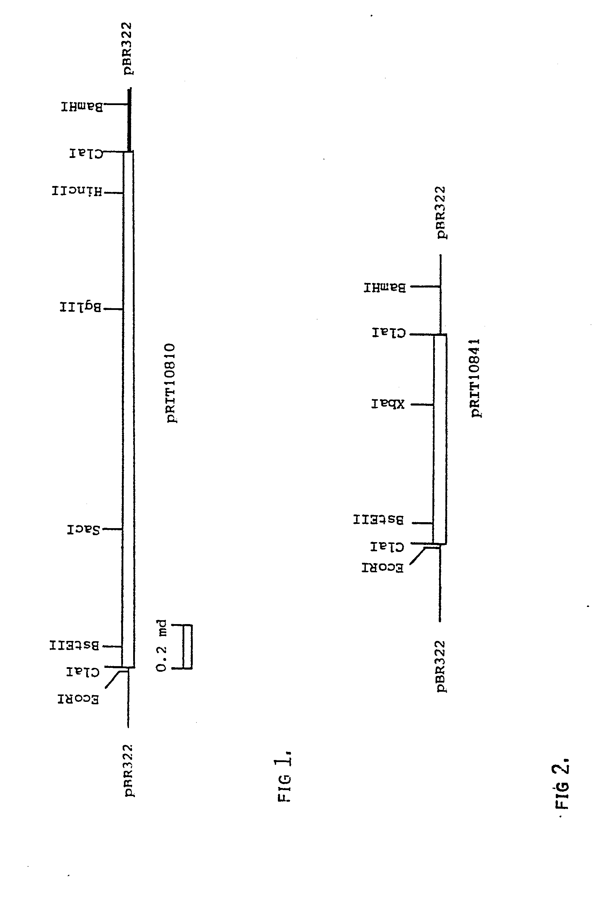

- on a cloné un fragment d'ADN de 1 x $10^6$ d, produit par ClaI, contenant la majeure partie du gène ctxA dans le plasmide pBR322, comme indiqué à la figure 1 qui représente la carte de restriction de l'insert de 1 x $10^6$ daltons dans le plasmide recombinant pRIT10841 ainsi obtenu.

- on a cloné un fragment d'ADN de 2,45 x $10^6$ d, produit par ClaI, contenant le gène ctxB dans le plasmide pBR322, comme indiqué à la figure 2 qui représente la carte de restriction de l'insert de 2,45 x $10^6$ daltons dans le plasmide recombinant pRIT10810 ainsi obtenu.

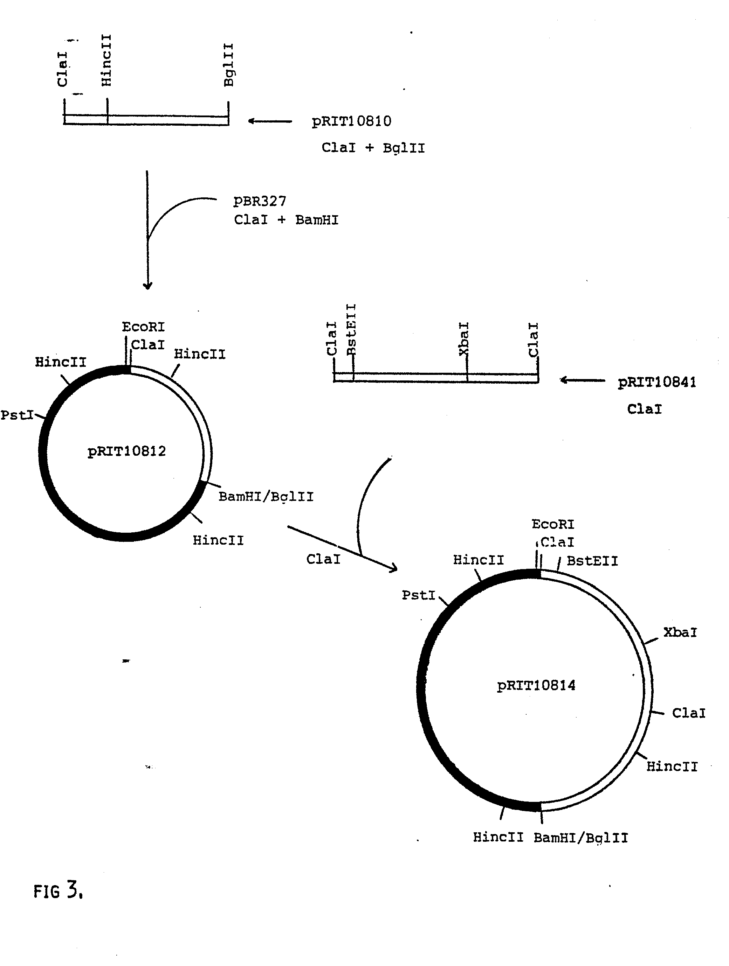

- on a cloné les fragments contenant les deux gènes ctxA et ctxB dans le plasmide pBR327 de façon à ce que l'insert ctxB de 0,75 x $10^6$ daltons produit par ClaI-BglII s'unisse à l'insert ctxA de 1 x $10^6$ d produit par ClaI, comme indiqué à la figure 3 qui représente la carte de restriction des inserts dans le plasmide recombinant pRIT10814 ainsi obtenu.

Des échantillons d'une souche de E. coli K12 ont été transformés, respectivement, par les plasmides pRIT 10841, pRIT 10810 et pRIT 10814 et des cultures de ces souches transformées ont été déposées à l'American Type Culture Collection, Rockville, Maryland, E.U.A. respectivement sous les numéros d'ordre ATCC 39053, ATCC 39051 et ATCC 39052.

De plus, on a cloné un fragment d'ADN de 5,1 x $10^6$ d produit par PstI à partir de l'ATCC 39050 dans le vecteur plasmidique pBR322 de façon à former le plasmide recombinant pRIT 10824. Ce plasmide possède le même nombre et la même disposition des sites de restriction à l'intérieur et autour des séquences de codage de ctx que le pRIT10814 susmentionné.

Il est évident que le procédé ci-dessus ne se limite pas à la souche ATCC 39050 exemplifiée et que, en utilisant à titre de produit de départ, une souche de V. cholerae produisant soit la seule sous-unité A ou la seule sous-unité B, on obtiendra des bandes d'ADN contenant respectivement soit le cistron ctxA soit le cistron ctxB.

Si on procède au clonage de l'ADN de la souche classique de Vibrio cholerae 569B (ATCC 25870) digéré par ClaI et contenant deux copies du gène de la toxine, on devra choisir des fragments de 1,25 x $10^6$ d pour représenter les séquences ctxA et des fragments de 0,88

x $10^6$ d et/ou 0,78 x $10^6$ d pour représenter les séquences ctxB.

Chacune des sous-unités A et B ou les deux ensemble s'avère(nt) précieuse(s) pour la préparation de vaccins anticholériques pour lesquels la sous-unité A est nécessairement utilisée sous la forme d'une antitoxine et la sous-unité B est, avec une préférence moindre, utilisée sous la forme d'une antitoxine.

Selon un autre mode de réalisation de la présente invention, on utilise les fragments d'ADN clonés pour produire les formes anatoxiques des sous-unités A ou B par manipulation génétique in vivo ou in vitro. Des exemples de manipulations bien connus des spécialistes sont la suppression ou l'insertion de paires de bases nucléotidiques au niveau des sites de restriction spécifiques ou la mutagénèse dirigée à des sites spécifiques afin de modifier des acides aminés particuliers. De plus, il est également possible de créer des molécules de toxine hybride par manipulation génétique in vitro, par exemple, par recombinaison du gène apparenté à l'entérotoxine thermolabile (TL) de E. coli avec le gène cloné de la toxine cholérique de l'invention au niveau de leurs sites de restriction XbaI homologues de façon à remplacer le site promoteur du gène de la toxine cholérique, le peptide directeur ou signal et les neuf premiers acides aminés de la protéine de la sous-unité A à maturité par les séquences TL correspondantes. L'expression d'une toxine hybride de ce genre se composant principalement de séquences de toxine cholérique devrait donc être réglée par un site promoteur de E. coli présentant des avantages pour la synthèse de la toxine dans les cellules de E. coli.

Les formes pharmaceutiques contenant soit la sous-unité B soit une anatoxine et précieuses comme vaccins à administrer par voie orale et/ou parentérale se préparent selon n'importe quelle technique connue des spécialistes pour une préparation de ce genre, c'est-à-dire, par exemple, par addition d'un antiacide tel que le bicarbonate de sodium ou par enrobage entérique ou par microencapsulation de l'ingrédient actif; l'anatoxine s'obtient également aisément à partir de la toxine, p.ex. par traitement au formaldéhyde ou, de préférence, au glutaraldéhyde.

Les sous-unités A et B suivant la présente invention s'avèrent également précieuses pour la caractérisation des souches de V. cholerae naturelles grâce à leur utilisation possible comme sondes radiomarquées au cours d'expériences d'hybridation ADN/ADN pour détecter des fragments de gènes ctxA et/ou ctxB dans des souches inconnues de V. cholerae par digestion de leur ADN total avec des endonucléases de restriction ou par hybridations de colonie.

L'invention est illustrée par les exemples suivants.

EXEMPLE 1

Préparation de l'ADN du V. cholerae ATCC 39050.

On laisse le V. cholerae ATCC 39050 se développer dans un milieu Syncase (FINKELSTEIN et coll. J. Immunol. 96, 440-449 (1966) jusqu'à atteindre la phase stationnaire. On récupère ensuite les cellules par centrifugation, on les lave et on les remet en suspension dans un tampon contenant de la tvométhamine(0,05 M), du NaCl (0,1 M) et de l'acide édétique (EDTA)(0,25 M), pH 8. On lyse les cellules par addition de dodécyl sulfate de sodium (SDS) jusqu'à concentration finale de 1 % (p/v) et on extrait le mélange à deux reprises avec des volumes égaux de phénol saturé en tampon. On précipite l'ADN à l'aide de deux volumes d'éthanol froid, on le récupère en l'enroulant sur une tige de verre et le redissout dans 1 x SSC (1 x SSC correspond au citrate salin standard, c'est-à-dire : 0,15 M en NaCl, 0,015 M en citrate trisodique, pH 7).

On ajoute le tampon trométhamine 0,05 M (pH 8) et on incube la préparation à l'aide de ribonucléase pancréatique bovine (concentration finale : 10 µg/ml) à 37°, pendant 1 heure. On extrait le mélange à deux reprises avec des volumes égaux de phénol saturé en tampon, on précipite l'ADN avec l'éthanol, on le dissout dans 1 x SSC et on le dialyse contre 1 x SSC.

On fait digérer un échantillon de 6 µg de l'ADN ainsi obtenu par 45,6 unités d'endonucléase ClaI dans un volume total de 40 µl, à 37° pendant 3 heures.

On divise l'ADN de Vibrio cholerae digéré par ClaI en deux échantillons égaux et on effectue l'électrophorèse dans des logettes séparées d'un gel d'agarose à 1 % (le tampon d'électrophorèse est celui décrit par HAYWARD G.S. et SMITH M.G., J. Mol. Biol. 63, 383 (1972) et contient 0,5 µg/ml de bromure d'éthidium).

On fait passer des échantillons d'ADN du phage lambda digérés séquentiellement par les endonucléases de restriction HindIII et EcoRI dans d'autres logettes du même gel de façon à ce qu'ils servent comme marqueurs de poids moléculaire en utilisant les valeurs fournies par CORY, S. et ADAMS, J. Cell 11; 795 (1977).

Après électrophorèse, on expose le gel à un rayonnement ultraviolet à onde courte de façon à visualiser les fragments d'ADN et on les photographie pour conserver un enregistrement de la migration relative des bandes d'ADN du phage lambda. On transfère ensuite les fragments d'ADN dans le gel et on les fixe sur un filtre en nitrocellulose par la technique de SOUTHERN E.M., J. Mol. Biol. 98, 503 (1975).

On découpe le filtre en deux dans le sens de la longueur pour obtenir les échantillons d'ADN de <u>Vibrio cholerae</u> digérés par ClaI sur chaque moitié du filtre.

<u>EXEMPLE 2</u>

<u>Préparation des sondes d'ADN spécifiques des gènes eltA et eltB</u>.

On prépare l'ADN du plasmide pEWD299 par centrifugation en gradient de densité de CsCl selon la méthode décrite par KAHN M. et coll., Methods in Enzymology, <u>68</u>, 268 (1979), on sépare les fragments d'ADN souhaités et on les purifie en faisant digérer 50 à 100 μg d'ADN du plasmide pEWD299 soit par un excès d'endonucléase de restriction HincII (pour <u>elt</u>A) soit, séquentiellement, par l'endonucléase de restriction HindIII et EcoRI (pour <u>elt</u>B) et électrophorèse subséquente sur gel d'acrylamide à 7,5 %. La construction et la caractérisation du plasmide pEWD299 est décrite par DALLAS W. et coll. dans J. Bact. <u>139</u>, 850 (1979).

On excise au scalpel la partie du gel qui contient le fragment souhaité (le fragment du gène <u>elt</u>A est un fragment d'ADN de 1000 paires de base et le fragment du gène <u>elt</u>B est un fragment d'ADN de 600 paires de base), on la place dans un sac de membrane de dialyse et on élue l'ADN de la tranche de gel par électrophorèse. On récupère l'ADN élué dans le tampon d'électrophorèse à l'intérieur du sac et on le concentre par précipitation à l'éthanol. On dissout l'ADN récupéré dans 20 μl de tampon trométhamine (0,01 M), pH 7.

Les fragments d'ADN spécifiques des gènes <u>elt</u>A et <u>elt</u>B ainsi obtenus sont ceux décrits par MOSELEY S. et FALKOW S., J. Bacteriol. <u>144</u>, 444 (1980).

EXEMPLE 3

Marquage des fragments de sonde d'ADN par déplacement de coupure (Nick-translation).

Des aliquotes (0,5 µg) de fragments d'ADN de eltA et eltB purifiés, tels que décrits à l'exemple 2, sont marquées dans des mélanges réactionnels séparés, à l'aide de désoxycytidine 5'-triphosphate ($\alpha$-$^{32}$P) par la méthode connue sous le nom de "Nick-translation" (RIGBY P. et coll., J. Mol. Biol. 113, 237 (1977) en utilisant la trousse (NEK-004) et les conditions de réaction fournies par la société New England Nuclear (Dreieich, République Fédérale d'Allemagne).

Les activités spécifiques des fragments d'ADN ainsi marqués sont : 2,3 x 10$^7$ cpm par 0,5 µg d'ADN pour l'ADN de eltA et 3,14 x 10$^7$ cpm par 0,5 µg d'ADN pour l'ADN de eltB. Avant utilisation, on dénature l'ADN marqué, par chauffage à 100° C pendant 10 à 15 minutes.

EXEMPLE 4

Hybridation de l'ADN fixé sur le filtre à l'aide de la sonde elt A.

Dans une solution de préhybridation contenant 5 x SSC, 1 mM EDTA pH 8, 0,1 % p/v de dodécyl sulfate de sodium (SDS), 1 % v/v de la solution décrite par DEN-HARDT D., Biochem. Biophys. Res. Comm. 23, 641 (1966) préparée sous forme d'une solution concentrée 100 x et 25 % v/v de formamide désionisé, on fait incuber à 37° pendant 3 heures une moitié du filtre de nitrocellulose contenant les fragments d'ADN fixés dénaturés de V. cholerae préparés comme décrit à l'exemple 1. On place ensuite le filtre dans une solution d'hybridation de même composition mais contenant en plus 25 µg par ml d'ADN de sperme de saumon dénaturé par la chaleur et on dilue la sonde eltA radioactive, dénaturée de l'exemple 3 de façon à obtenir une activité spécifique finale

de $9 \times 10^5$ cpm par ml. On incube le filtre dans cette solution à 37° pendant 36 heures et on l'incube ensuite à 37° à deux reprises pendant 25 minutes dans des solutions de même composition que celles utilisées pour l'hybridation mais sans l'ADN de la sonde radioactive. On rince ensuite le filtre à deux reprises pendant 25 minutes à température ambiante dans des solution de 2 x SSC et on le sèche à l'air. On expose le filtre séché à un film radiologique (Fuji Photo film Co. Ltd) pendant une durée suffisante de façon à noircir les surfaces du film correspondant aux surfaces du filtre où l'ADN de la sonde radioactive s'est hybridé aux fragments dénaturés et fixés de l'ADN de V. cholerae.

L'examen des films radiologiques exposés au filtre incubé avec la sonde eltA indique que le fragment du gène eltA s'hybride spécifiquement et exclusivement avec une seule bande de l'ADN total du V. cholerae ATCC 39050 digéré par ClaI. Ce fragment d'ADN a un poids moléculaire estimé à $1 \times 10^6$ daltons par rapport à la migration relative des fragments d'ADN de lambda. La séquence d'ADN de V. cholerae qui, dans ces conditions d'hybridation, montre une homologie à l'égard de la sonde eltA représente la totalité ou une partie du cistron ctxA lequel détermine la séquence du gène de structure pour la sous-unité A de la toxine cholérique.

EXEMPLE 5

Hybridation de l'ADN fixé sur le filtre avec la sonde eltB.

Pour hybrider l'ADN de la sonde eltB, on suit un procédé identique avec l'autre moitié du filtre qui contient l'ADN dénaturé de V. cholerae excepté que l'on utilise une concentration de 20 % v/v en formamide et on dilue l'ADN de la sonde eltB de façon à obtenir une activité spécifique de $1,2 \times 10^6$ cpm par ml dans la solution d'hybridation. La sonde eltB s'hybride spécifiquement et exclusivement dans les conditions décrites avec une seule bande d'ADN de V. cholerae ATCC 39050 digéré par ClaI et d'un poids moléculaire estimé à 2,45 x $10^6$ daltons.

La séquence d'ADN de V. cholerae qui, dans ces conditions d'hybridation, montre une homologie à l'égard de la sonde eltB représente la totalité ou une partie du cistron ctxB lequel détermine la séquence du gène de structure pour la sous-unité B de la toxine cholérique.

## EXEMPLE 6

Enrichissement des fragments d'ADN contenant soit les séquences du gène ctxA, soit les séquences du gène ctxB à partir de l'ADN total du V. cholerae.

Une aliquote de 63 µg de l'ADN total telle qu'obtenue à l'exemple 1 est mise à digérer par 500 unités d'endonucléase ClaI à 37° pendant 3 heures, dans un volume réactionnel total de 400 µl. On effectue l'électrophorèse de l'ADN digéré sur un gel d'agarose à 1 % (p/v). On fait passer un échantillon d'ADN du phage lambda digéré par les endonucléases EcoRI et HindIII et un échantillon d'ADN de pEWD020 (W. DALLAS et coll., J. Bacteriol. 139, 850 (1979)) digéré par les endonucléases BamHI et EcoRI dans des logettes séparées du même gel de façon à ce qu'ils servent comme marqueurs de poids moléculaire. On évalue la position des fragments d'ADN de V. cholerae, à partir de la digestion avec l'endonucléase ClaI, correspondant à des poids moléculaires de 2,45 x $10^6$ d et 1 x $10^6$ d en se référant aux marqueurs de poids moléculaire et on découpe cinq tranches d'1 mm en travers du gel au-dessus et en dessous de chacun de ces deux points. On récupère l'ADN dans chaque tranche de gel par électroélution dans un sac fermé d'une membrane de dialyse. On élimine le liquide de chaque sac, on le filtre à travers un filtre Millipore de 0,45 μm de façon à éliminer les fragments d'agarose et on récupère l'ADN par précipitation à l'aide d'un volume égal d'isopropanol. On dissout le précipité dans un tampon trométhamine (0,01 M) à pH 7 et on le reprécipite à l'aide de 2 volumes d'éthanol. On dissout finalement l'ADN obtenu à partir de chaque tranche de gel dans 20 μl d'une solution tampon trométhamine (0,01 M), EDTA (0,001 M), à pH 7.

On analyse des échantillons de un μl de chaque préparation d'ADN pour leur contenu en séquences de gène ctxA ou ctxB selon la méthode d'hybridation ponctuelle décrite par KAFATOS F. et coll. (Nucl. Acids Res. 7, 1541 (1979)) et en utilisant les conditions d'hybridation et les ADN des sondes eltA et eltB radioactives décrites dans les exemples 2 à 5.

On trouve ainsi deux préparations d'ADN correspondant à de l'ADN de 1 x $10^6$ d qui s'hybrident plus fortement que les trois autres avec la sonde eltA .

De même, deux préparations d'ADN de 2,45 x $10^6$ d en ADN s'hybrident plus fortement avec la sonde eltB.

## EXEMPLE 7

### Clonage d'ADN enrichi pour les séquences du gène ctxB.

On fait digérer des aliquotes (5 μg) d'ADN du plasmide pBr322, préparé par centrifugation en gradient de concentration de CsCl, par 6,6 unités d'endonucléase ClaI dans un volume réactionnel de 100 μl, à 37° pendant 2 heures. A 60 μl d'échantillon de cette digestion contenant 3 μg d'ADN de plasmide, on ajoute 100 μl de tampon glycine (glycine (0,1 M); $MgCl_2$, $6H_2O$ (1 mM); $ZnSO_4$, $7H_2O$ (0,1 mM)) à pH 10,5) et 0,3 unité de phosphatase alcaline d'intestin de veau. On fait incuber le mélange à 37° pendant 30 minutes, on l'extrait à deux reprises par un volume égal de phénol, on l'extrait trois fois à l'éther et on précipite l'ADN avec de l'éthanol. On dissout l'ADN de pBR322 dans 30 µl' de tampon trométhamine-HCl (0,01 M) EDTA (0,001 M) à pH 7.

On mélange des aliquotes (10 µl) de chacune des deux fractions d'ADN obtenues à l'exemple 6 et montrant le signal d'hybridation le plus prononcé à l'égard de la sonde eltB avec 0,4 µg d'ADN de pBR322 digéré par ClaI, traité par la phosphatase alcaline et on amène le mélange à un volume final de 30 µl à l'aide du tampon trométhamine, on l'extrait avec un volume égal de phénol et ensuite trois fois à l'éther, on le précipite avec de l'éthanol et on dissout l'ADN dans 20 µl de tampon trométhamine-HCl (0,01 M); EDTA (0,001 M) à pH 7.

On ligue le mélange des fragments d'ADN de V. cholerae et de l'ADN de pBR322 par incubation à l'aide de l'ADN ligase de T4. A 10 µl d'un mélange d'ADN, on ajoute 10 µl d'un cocktail d'ADN ligase de T4 de façon à obtenir une solution 20 mM en trométhamine-HCl pH 7,6, 10 mM en $MgCl_2$ $6H_2O$, 10 mM en dithiothreitol, 1,2 mM en triphosphate de désoxyadénosine et contenant 50 µg par ml de sérum-albumine de boeuf et 0,5 unité d'ADN ligase de T4 (Boehringer Mannheim, Républ. Féd. d'Allemagne).

On incube le mélange contenant la ligase à 15° C pendant 4 heures de façon à obtenir la ligation des fragments d'ADN du V. cholerae à l'ADN du pBR322 digéré par ClaI et former les molécules recombinantes.

On utilise l'entièreté du mélange d'ADN ligué (20 µl) pour la transformation des cellules compétentes traitées au $CaCl_2$ de la souche MM294 de E. coli K12 (décrite par BACKMAN K. et coll., Proc. Natl. Acad. Sci. U.S. 73, 4174 (1976)) préparées selon le procédé décrit par COHEN S. et coll., Proc. Natl. Acad. Sci. U.S. 69, 2110 (1972)). On disperse la culture transformée sur un milieu de gélose solide contenant 200 µg d'ampicilline par ml de façon à sélectionner les transformants en fonction des cellules qui ont capté l'ADN du plasmide pBR322. De l'ensemble de la culture transformée, on récupère approximativement 1000 colonies résistantes à l'ampicilline.

### EXEMPLE 8

### Criblage des colonies transformées pour les séquences du gène ctxB.

On sélectionne les colonies transformées résistantes à l'ampicilline, obtenues à l'exemple 7, en fonction de la présence des séquences d'ADN s'hybridant avec la sonde eltB, selon la méthode d'hybridation de colonie décrite par GERGEN J. et al., Nucl. Acids Res. 7, 2115 (1979). Les colonies de transformants poussant sur le milieu de gélose solide sont transférées à la surface de plaques dédoublées de milieu contenant de l'ampicilline et on incube les plaques à 37°. On place un carré de papier filtre stérile (Whatman 541) à la surface d'une plaque de chaque paire et on enlève la prolifération bactérienne de la surface gélosée en écartant le papier filtre. Par ce procédé, on obtient une réplique sur papier filtre des colonies poussant sur le milieu de gélose solide. On sèche les papiers-filtres à 37° pendant 15 minutes et on procède de la façon décrite par GERGEN J. et coll., (loc. cit.) excepté que l'on omet le lavage à l'éthanol.

On hybride les papiers filtres ainsi traités avec l'ADN de la sonde radioactive eltB dans des conditions exactement identiques et selon le procédé décrit à l'exemple 5 et on les expose à un film radiologique pendant des périodes de temps variables.

On note que dix colonies produisent un noircissement plus important que les autres, on les localise et on les récupère des plaques de gélose dédoublées. De ces 10 colonies présentant un résultat d'hybridation positif, quatre s'avèrent sensibles à la tétracycline, ce qui dénote l'inactivation de l'insertion du gène de résistance à la tétracycline sur le vecteur pBR322 par un fragment d'ADN étranger.

On prépare l'ADN plasmidique à partir de chacune de ces quatre colonies selon le procédé de BIRNBOIM H. et DOLY J. (Nucl. Acids Res. $\underline{7}$, 1513 (1979) et l'analyse par l'endonucléase de restriction ClaI montre que dans chaque préparation le vecteur pBR322 porte un insert d'ADN de poids moléculaire 2,45 x $10^6$ d. On caractérise une colonie ainsi isolée comme MM294 (pRIT10810) dont une culture a été déposée à l'American Type Culture Collection sous le numéro d'ordre ATCC 39051.

## EXEMPLE 9
### Caractérisation de la souche ATCC 39051.

On prépare l'ADN plasmidique à partir de la souche ATCC 39051 par centrifugation en gradient de densité de CsCl-bromure d'éthidium et on le soumet à une nouvelle analyse par endonucléase de restriction. L'insert d'ADN ClaI de 2,45 x $10^6$ daltons est caractérisé par la possession des sites de restriction et l'orientation vis-à-vis de l'ADN du vecteur pBR322 comme l'indique la figure 1.

On caractérise davantage la souche par la production d'une protéine qui réagit avec des anticorps dressés contre la toxine cholérique pure selon une méthode dite ELISA décrite par SVENNERHOLM A. et HOLMGREN J. (Curr. Microbiol. $\underline{1}$, 19, 1978) et basée sur la fixation de la protéine de la sous-unité B de la toxine cholérique sur les récepteurs gangliosidiques de type GM1.

On constate que les extraits de cellules cultivées sur bouillon de la souche ATCC 39051 préparés par passage à travers une cellule à pression de French et cla-

rifiés par centrifugation à 30.000 G à 4° C pendant 30 minutes sont fortement positifs à l'égard de la protéine de la sous-unité B selon cette méthode ELISA.

Afin de caractériser davantage l'insert cloné, on fait digérer l'ADN de pRIT10810 séquentiellement par les endonucléases ClaI et HincII et on purifie le fragment $0,2 \times 10^6$ d par électrophorèse sur un gel d'acrylamide et électroélution de la tranche de gel contenant le fragment. On marque 0,5 µg de ce fragment purifié à l'aide de $^{32}$P par "Nick translation", comme décrit à l'exemple 3, jusqu'à obtention d'une activité spécifique de $1,96 \times 10^8$ cpm par 0,5 µg pour l'utiliser comme sonde. On fait digérer l'ADN total de la souche ATCC 39050 par différentes endonucléases et on procède à l'électrophorèse des échantillons sur gels d'agarose, on les dénature et on les transfère sur des filtres de nitrocellulose. On incube ces filtres avec l'ADN de la sonde ClaI-HincIII radioactive, dénaturée dans des conditions d'hybridation ADN/ADN strictes, en excluant le formamide des solutions d'hybridation et en effectuant l'incubation à 65° pendant 18 heures.

L'examen des films radiologiques exposés aux filtres de nitrocellulose hybridés indique qu'on révèle exactement les mêmes bandes d'ADN de <u>Vibrio</u> avec là sonde ClaI-HincII de <u>Vibrio</u> que celles qu'on a détectées dans l'exemple 5 avec la sonde <u>eltB</u> dans des conditions moins strictes. Ce résultat montre que l'ADN cloné provient du <u>Vibrio cholerae.</u>

<u>EXEMPLE 10</u>

<u>Clonage d'ADN de V. cholerae enrichi pour les séquences du gène ctxA.</u>

On mélange dix µg de chacune des deux fractions d'ADN digérées par ClaI (manifestant le signal d'hybridation le plus prononcé envers la sonde <u>eltA</u>), décrite

- 17 -

0095452

à l'exemple 7, avec 0,4 µg d'ADN de pBR322 digéré par ClaI et traité par la phosphatase alcaline.

On extrait le mélange d'ADN au phénol, on le précipite à l'alcool, on le dissout, on le ligue à l'aide d'ADN ligase de T4 exactement comme à l'exemple 7 et on l'utilise pour transformer les cellules traitées au CaCl$_2$ de la souche MM294 de E. coli K12. On récupère un total d'environ 4000 colonies par inoculation de la culture transformée sur milieu gélosé contenant de l'ampicilline à 37°.

On sélectionne les colonies résistantes à l'ampicilline décrites ci-dessus en fonction de la présence des séquences d'ADN s'hybridant à la sonde eltA en utilisant les procédés et conditions décrits à l'exemple 8. On détecte deux colonies qui donnent une réponse positive avec la sonde eltA et qui sont sensibles à la tétracycline. On prépare l'ADN plasmidique à partir de ces deux clones par la méthode de BIRNBOIM et DOLY (loc. cit.) et la digestion par l'endonucléase ClaI libère, en plus du fragment du vecteur pBR322, un fragment d'ADN de 1 x 10$^6$ d. On caractérise les deux colonies comme étant MM294 (pRIT10841) et MM294 (pRIT10851). Une culture de MM294 (pRIT10841) a été déposée à l'American Type Culture Collection sous le numéro d'ordre ATCC 39053.

### EXEMPLE 11

#### Caractérisation de la souche ATCC 39053.

On prépare l'ADN plasmidique à partir de la souche ATCC 39053 par centrifugation en gradient de CsCl-bromure d'éthidium et on le soumet à une nouvelle analyse par endonucléase de restriction. L'insert d'ADN de 1 x 10$^6$ daltons est caractérisé par la possession des sites de restriction BstEII et XbaI et l'orientation vis-à-vis de l'ADN du vecteur pBR322 telles qu'indiquées dans la figure 2. L'ADN de l'insert ClaI est caractérisé davantage par l'absence de possession de sites pour les endonucléases EcoRI, BamHI, HindIII, PstI, SacI, PvuI, XhoI, AvaI, BglII, HpaI, SmaI, HincII, PvuII ou SalI.

On fait digérer de l'ADN purifié de pRIT10841 par l'endonucléase ClaI et on purifie le fragment de 1 x $10^6$ daltons par électrophorèse en gel d'agarose et électroélution. On marque 0,5 µg de cet ADN au $^{32}$P par "Nick Translation" comme décrit à l'exemple 3 et on l'utilise comme sonde.

On fait digérer de l'ADN total de la souche ATCC 39050 par diverses endonucléases de restriction et on procède à l'électrophorèse des échantillons sur gels d'agarose, on les dénature in situ et on les transfère sur des filtres de nitrocellulose. L'incubation, dans des conditions d'hybridation rigoureuses, du fragment d'ADN de la sonde ClaI dénaturée avec l'ADN de Vibrio fixé sur les filtres indique que la sonde détecte exactement les mêmes bandes d'ADN de Vibrio que celles qui ont été détectées par la sonde eltA dans des conditions d'hybridation non rigoureuses ("relaxed").

Ce résultat indique que l'ADN cloné provient du Vibrio.

EXEMPLE 12

Construction du pRIT10812.

Une aliquote de 25 µg d'ADN plasmidique de pRIT10810 préparé comme décrit à l'exemple 9 est mise à digérer séquentiellement par 55 unités d'endonucléase ClaI à 37° pendant 3 heures et 24 unités d'endonucléase BglII à 37° pendant 2 heures. On procède à l'électrophorèse de l'ADN digéré sur un gel d'agarose à 1 % et on excise le fragment d'ADN ClaI-BglII contenant les séquences du gène ctxB à partir du gel et on récupère l'ADN par électroélution.

On fait digérer 2,5 µg d'ADN plasmidique de pBR327 (SOBERON X. et coll., Gene 9, 287, 1980) séquentiellement par 11 unités d'endonucléase ClaI à 37° pendant 2,5 heures et 8 unités d'endonucléase BamHI à 37° pendant 2 heures. L'ADN digéré est extrait à deux reprises au phénol et trois fois à l'éther, on le précipite à l'éthanol et on le dissout dans un tampon trométhamine (0,01 M) à pH 7. On ligue une aliquote de 0,4 µg du fragment d'ADN ClaI-BglII purifié du ctxb à l'aide de 0,2 µg de l'ADN de pBR327 digéré par ClaI et BamHI dans les conditions de ligation décrites cidessus.

On utilise le mélange d'ADN formé par ligation pour transformer les cellules traitées au $CaCl_2$ de la souche MM294 de E. coli K12. On sélectionne les transformants sur un milieu gélosé solide contenant200 µg/ml d'ampicilline.

Une des colonies ainsi isolée montre qu'elle contient un plasmide, le pRIT10812, qui se compose en majeure partie du fragment ClaI-BamHI du pBR327 et d'un insert de fragment ClaIBglII purifié à partir du pRIT10810. Le plasmide est caractérisé par la possession de la carte de restriction indiquée à la figure 3.

EXEMPLE 13

Combinaison du fragment ctxA et ctxB - Construction du pRIT18014.

Une aliquote de 51 µg d'ADN plasmidique de pRIT10841 purifié, préparé comme décrit à l'exemple 11 est mise à digérer par 27 unités d'endonucléase ClaI à 37° pendant 18 heures. On procède à l'électrophorèse du mélange sur un gel d'agarose à 1 % et on excise une tranche de gel contenant le fragment d'ADN ClaI de 1 x $10^6$ daltons.

On récupère l'ADN de la tranche de gel par électroélution et précipitation à l'éthanol.

Une aliquote de 2,86 µg d'ADN purifié de pRIT-10812, préparé comme décrit à l'exemple 12 est mise à digérer par 11 unités d'endonucléase ClaI à 37° pendant 2 heures et on la traite par 0,25 unité de phosphatase alcaline d'intestin de veau, comme décrit à l'exemple 7. On extrait l'ADN ainsi traité au phénol et on le précipite à l'éthanol. On ligue une aliquote de 0,4 µg de cet ADN à de 0,25 µg de fragment d'ADN ClaI purifié isolé du pRIT10841 dans les conditions de ligation décrites à l'exemple 7 ci-dessus. On utilise la moitié du mélange d'ADN ligué pour transformer les cellules compétentes traitées au $CaCl_2$ de la souche MM294 de E. coli K12, la sélection étant effectuée avec de l'ampicilline (200 µg/ml) sur un milieu d'agar solide. On prépare l'ADN plasmidique selon la méthode de BIRNBOIM et DOLY (loc. cit.) à partir de 12 colonies et on le restreint séquentiellement à l'aide des endonucléases PstI et XbaI. Un plasmide ainsi obtenu donne un motif de fragment de restriction qui dénote l'insertion d'un fragment ClaI de 1 x $10^6$ daltons sur le pRIT10812 dans l'orientation désirée et il est caractérisé plus amplement.

On prépare l'ADN plasmidique à partir du clone par centrifugation sur CsCl-bromure d'éthidium.

L'ADN plasmidique du clone MM294(pRIT10814) révèle à l'analyse par endonucléase de restriction qu'il possède la structure indiquée à la figure 3. Une culture de MM294(pRIT10814) a été déposée à l'American Type Culture Collection sous le numéro d'ordre ATCC 39052.

Les étapes de combinaison susmentionnées sont schématisées dans la figure 3.

On conclut que l'orientation du fragment d'ADN ClaI de 1 x $10^6$ daltons à partir du pRIT10841 dans le plasmide recombinant pRIT10814 est telle qu'elle juxtapose la séquence ctxA à la séquence ctxB au site ClaI,

c'est-à-dire qu'elle reforme l'entièreté des cistrons qui déterminent le gène de structure pour la toxine cholérique.

EXEMPLE 14

Activité in vivo de la souche ATCC 39052.

Dans les anses intestinales ligaturées de dix lapins adultes, on injecte des échantillons d'un ml d'extraits de cellules de la souche ATCC 39052. Ces extraits sont préparés comme décrit à l'exemple 9, et contiennent 10 à 13 mg/ml de protéine totale. On examine les anses 18 heures plus tard et on mesure l'accumulation de liquide dans chaque anse.

Considérant qu'un rapport $\leqslant 0,5$ signifie une réponse négative et qu'un rapport $\geqslant 0,5$ signifie une réponse positive, il ressort du tableau I que les extraits de la souche ATCC 39052 donnent une réponse positive chez neuf animaux sur dix.

Par contre, les extraits de la souche MM294(pBR322) ne donnent aucune accumulation de liquide mesurable chez cinq animaux testés. L'injection de 100 ng de toxine cholérique purifiée par anse engendre une réponse positive chez huit lapins sur huit, l'injection de 10 ng de toxine purifiée produisant le même effet chez trois lapins sur huit. Les extraits de la souche ATCC 39051 (pRIT10810) et de la souche ATCC 39053 (pRIT10841) s'avèrent également négatifs dans les épreuves, respectivement sur quatre et cinq lapins. Ce résultat démontre que les extraits de la souche ATCC 39052 (pRIT10814) contiennent une substance capable d'induire une accumulation de liquide dans l'intestin du lapin comme l'induit la toxine cholérique. Les cistrons ctxA et ctxB sur le pRIT10814 doivent par conséquent avoir été correctement reformés par ligation à leur site ClaI commun de façon à restaurer un gène de structure fonctionnelle déterminant la synthèse de
l'holotoxine cholérique.

Les résultats sont donnés dans le tableau I.

TABLEAU I

Epreuve de l'anse intestinale chez le lapin adulte.

| Extrait | Nombre de lapins testés | Nombre donnant une réponse positive | Rapport moyen : volume de liquide par cm d'anse intestinale |
|---|---|---|---|
| Souche MM294 | | | |
| (pBR322) | 5 | 0 | 0,1 |
| ATCC 39052 | 10 | 9 | 1,40 |
| ATCC 39051 | 4 | 0 | 0,1 |
| ATCC 39053 | 5 | 0 | 0,1 |
| Toxine cholé- | | | |
| rique | | | |
| 100 ng/ml | 8 | 8 | 1,62 |
| 10 ng/ml | 8 | 3 | 0,66 |

EXEMPLE 15

Clonage d'un fragment d'ADN PstI contenant les deux
cistrons ctxA et ctxB du V. cholerae ATCC 39050.

Afin de vérifier l'état complet des fragments
d'ADN ClaI clonés encodant les cistrons ctxA et ctxB,
on clone directement, à partir de la souche ATCC 39050,
un fragment d'ADN contenant les deux cistrons. L'analyse de l'ADN total de l'ATCC 39050 digéré par l'endonucléase PstI à l'aide des sondes eltA et eltB en utilisant les méthodes et procédés décrits ci-dessus dans
les exemples 1 à 5 démontre que les deux cistrons ctxA
et ctxB sont présents sur un ou des fragment(s) d'ADN
de poids moléculaire calculé de $5,3 \times 10^6$ D.

On enrichit ce fragment à partir d'une digestion
de 50 µg d'ADN de l'ATCC 39050 par 100 unités d'endonucléase PstI, selon les méthodes et procédés décrits à
l'exemple 6 et on ligue une aliquote de 6 µl de la

fraction montrant la réponse d'hybridation la plus prononcée avec la sonde _ctx_B ClaI-HindII de l'ADN de _V. cholerae_ décrite à l'exemple 9, avec 0,4 µg d'ADN plasmidique pBR322 préalablement digéré par PstI. Ce mélange d'ADN formé par ligation est transformé dans la souche MM294 de _E. coli_ K12, les colonies de transformants étant sélectionnées sur milieu d'agar solide complété avec 15 µg/ml de tétracycline.

On transfère ces colonies (environ 14000 au total) sur des papiers filtres Whatman 541, on traite les filtres comme décrit à l'exemple 8 et on les hybride avec la sonde du fragment _ctx_A radioactif décrite à l'exemple 11.

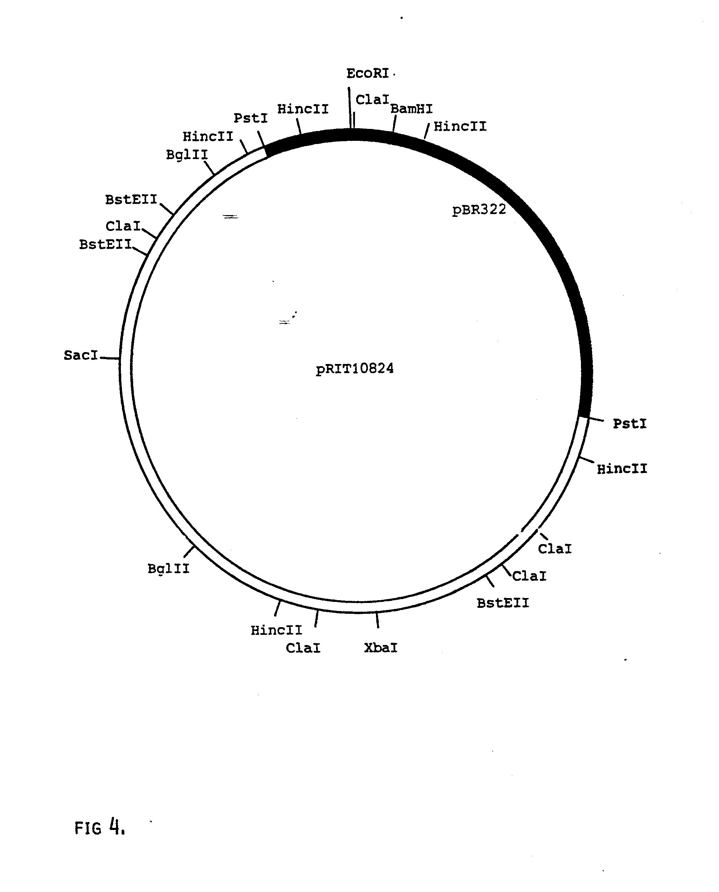

On détecte onze colonies qui montrent une réponse d'hybridation positive envers la sonde _ctx_A et qui s'avèrent résistantes à la tétracycline et sensibles à l'ampicilline, signe de l'insertion d'un fragment d'ADN étranger au site PstI du vecteur pBR322. On prépare l'ADN plasmidique à partir d'un de ces clones - la souche MM294(pRIT10824) - et on le soumet à l'analyse par enzyme de restriction. Le plasmide pRIT10824 contient un insert de $5,1 \times 10^5$ d au site PstI du vecteur et la digestion par l'endonucléase ClaI libère 5 fragments dont un correspond, par la taille, au fragment _ctx_B de $1 \times 10^6$ d précédemment cloné dans RIT10810 et un au fragment _ctx_A de $2,45 \times 10^6$ d précédemment cloné dans pRIT10841. De plus, ces fragments ClaI de $1 \times 10^6$ d et $2,45 \times 10^6$ d à partir du pRIT10824 possèdent le même nombre et la même localisation des sites de restriction que les fragments clonés séparément dans pRIT 10810 et pRIT10841. En outre, la comparaison des digestions d'enzyme de restriction entre le pRIT10824 et le pRIT10841 décrit à l'exemple 13 ne démontre aucune différence dans le nombre de fragments de restriction pour les enzymes ou combinaisons d'enzymes incisant dans ou autour des cistrons ctx. Une carte de restriction du pRIT10824 est donnée à la figure 4.

<div align="center">EXEMPLE 16</div>

Séquences d'ADN des cistrons ctxA et ctxB.

On détermine la séquence nucléotidique de l'ADN correspondant aux cistrons ctxA et ctxB selon la méthode de modification chimique de MAXAM et GILBERT en utilisant les procédés décrits dans Methods in Enzymology, 65, 499-560, 1980. On marque les fragments de restriction au $^{32}$P à leur extrémité 5' soit par kination d'échange ou par kination directe des extrémités déphosphorylées. Comme alternative, on marque les extrémités 3'en utilisant la transférase terminale et la $\alpha-^{32}$P-cordycépine (C.P. TU et S.N. COHEN, Gene, 10, 177-183, 1980). On obtient les fragments marqués à une seule extrémité par clivage à l'aide d'une autre endonucléase de restriction et purification sur gel de polyacrylamide. On utilise les réactions au sulfate de diméthyle, à l'hydrazine et à l'hydroxyde de sodium pour le clivage, respectivement aux niveaux guanine, pyrimidine et adénine. Après clivage au niveau pipéridine, on sépare les produits sur de minces gels à 8 % ou 20 % en polyacrylamide (SANGER & COULSON, FEBS Lett. 87, 107, 1978). On lit ensuite la séquence à partir de l'autoradiogramme du gel.

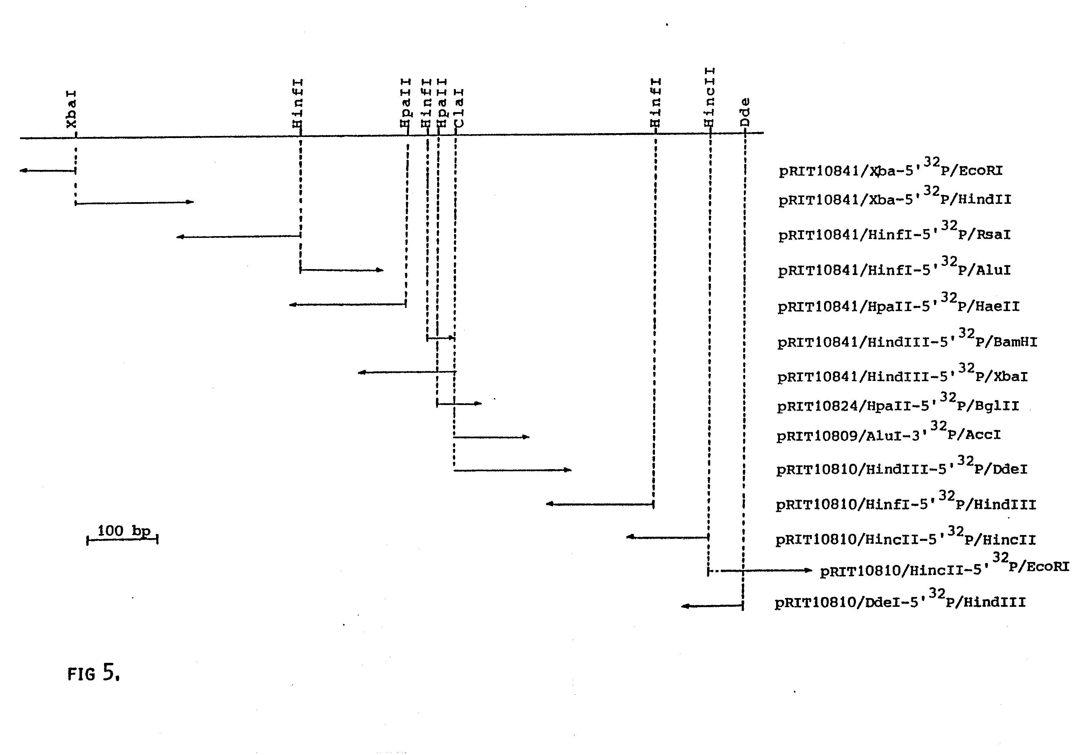

Afin de garantir le degré de précision le plus élevé dans la détermination de la séquence, on lit, à partir des différentes extrémités marquées, les séquences d'ADN qui se chevauchent et on détermine les séquences des deux brins d'ADN là où cela est possible. Les plasmides recombinants, les sites de restriction choisis pour le marquage et l'étendue de la séquence lue pour chaque détermination sont présentés à la figure 5.

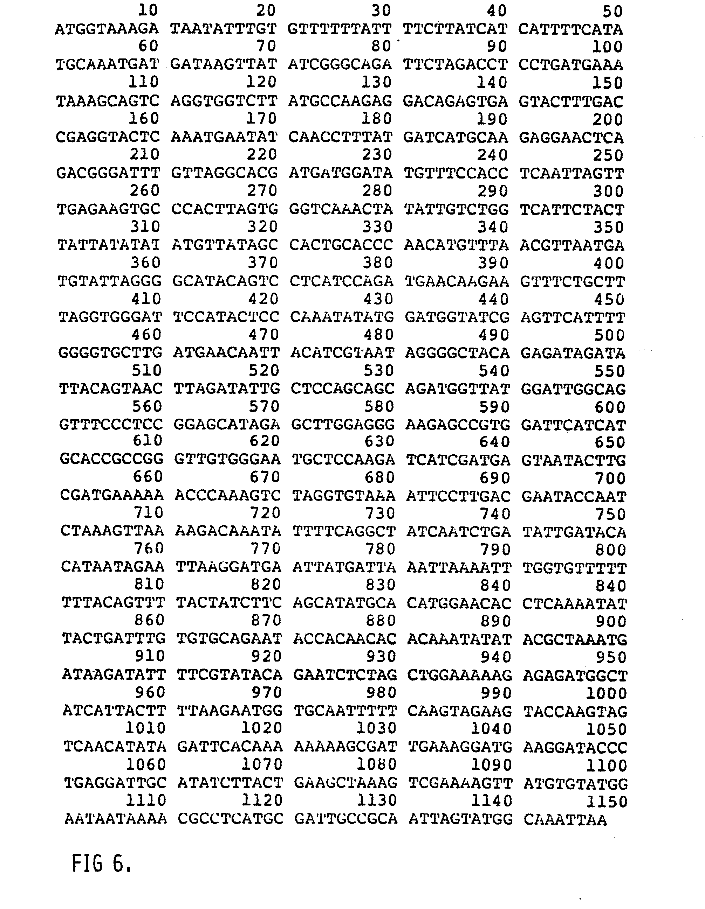

La séquence d'ADN dérivée de ces méthodes est présentée à la figure 6 et elle comprend 1148 nucléotides dont les nucléotides 1 à 777 inclus constituent la séquence de codage pour la sous-unité A et les nucléotides 774 à 1148 inclus constituent la séquence de codage pour la sous-unité B. Il est important que, dans la séquence en question, il existe des régions étendues qui correspondent aux séquences en acides aminés connues pour les protéines de la sous-unité A et sous-unité B de l'entérotoxine thermolabile de E. coli (cfr. SPICER E. et coll., Proc. Natl. Acad. Sci. US 78, 50, 1981) à l'exception bien entendu des erreurs et omissions dans les déterminations des séquences protéiniques de ce genre. Il faut également savoir que des variations en codons et acides aminés peuvent exister entre les différentes souches de Vibrio cholerae à la fois pour les sous-unités A et B et que la présente invention n'est pas limitée à la séquence décrite ci-dessous mais qu'elle comprend tous les équivalents fonctionnels de la séquence décrite ci-dessus. Ceci est exemplifié par comparaison de la séquence protéinique de la sous-unité B pour la souche Vibrio El Tor en question et de la séquence connue en acides aminés déterminée par LAI (J. Biol. Chem. 252, 7249, 1977) pour la protéine B de la souche 569B (ATCC 25870) de Vibrio classique où il existe cinq différences en acides aminés, notamment aux positions 18, 22, 47, 54 et 70 dans la séquence en acides aminés à maturité.

Il faut également noter que la séquence codante de la protéine de la sous-unité A traverse le site ClaI en marquant l'extrémité du fragment du gène ctxA en s'y prolongeant et en se terminant au nucléotide 777 à l'intérieur du fragment du gène ici appelé ctxB.

EXEMPLE 17

Préparation vaccinale

On laisse une souche de E. coli ATCC 39051 se développer dans un milieu Syncase (loc. cit.) jusqu'à atteindre la phase stationnaire. On centrifuge ensuite la culture et on la filtre, on concentre le filtrat qui en résulte, on le dialyse et on l'adsorbe sur de l'hydroxyde d'aluminium. Après lavage, on élue la solution de la sous-unité B de la toxine cholérique avec du citrate de sodium (0,1 M), on la tamponne à pH 7,2 à l'aide d'un tampon phosphate, on la répartit dans des flacons de 2 ml contenant 200 unités d'antitoxine par ml et on la lyophilise.

On utilise les flacons fraîchement réhydratés pour l'administration par voie orale, le schéma de vaccination comprenant une administration de rappel 30 jours après la première vaccination, chaque administration étant précédée de l'administration par voie orale de 2 g de bicarbonate de sodium dans 60 ml d'eau.

REVENDICATIONS POUR LES ETATS CONTRACTANTS

BE, CH, DE, FR, GB, IT, LI, LU, NL, SE.

1. Séquence d'ADN comprenant au moins une portion codant pour la totalité ou une partie des sous-unités A et B de la toxine cholérique.

2. Séquence d'ADN choisie dans le groupe comprenant en substance les séquences d'ADN de formule

```
         10        20        30        40        50
ATGGTAAAGA TAATATTTGT GTTTTTTATT TTCTTATCAT CATTTTCATA
         60        70        80        90       100
TGCAAATGAT GATAAGTTAT ATCGGGCAGA TTCTAGACCT CCTGATGAAA
        110       120       130       140       150
TAAAGCAGTC AGGTGGTCTT ATGCCAAGAG GACAGAGTGA GTACTTTGAC
        160       170       180       190       200
CGAGGTACTC AAATGAATAT CAACCTTTAT GATCATGCAA GAGGAACTCA
        210       220       230       240       250
GACGGGATTT GTTAGGCACG ATGATGGATA TGTTTCCACC TCAATTAGTT
        260       270       280       290       300
TGAGAAGTGC CCACTTAGTG GGTCAAACTA TATTGTCTGG TCATTCTACT
        310       320       330       340       350
TATTATATAT ATGTTATAGC CACTGCACCC AACATGTTTA ACGTTAATGA
        360       370       380       390       400
TGTATTAGGG GCATACAGTC CTCATCCAGA TGAACAAGAA GTTTCTGCTT
        410       420       430       440       450
TAGGTGGGAT TCCATACTCC CAAATATATG GATGGTATCG AGTTCATTTT
        460       470       480       490       500
GGGGTGCTTG ATGAACAATT ACATCGTAAT AGGGGCTACA GAGATAGATA
        510       520       530       540       550
TTACAGTAAC TTAGATATTG CTCCAGCAGC AGATGGTTAT GGATTGGCAG
        560       570       580       590       600
GTTTCCCTCC GGAGCATAGA GCTTGGAGGG AAGAGCCGTG GATTCATCAT
        610       620       630       640       650
GCACCGCCGG GTTGTGGGAA TGCTCCAAGA TCATCGATGA GTAATACTTG
        660       670       680       690       700
CGATGAAAAA ACCCAAAGTC TAGGTGTAAA ATTCCTTGAC GAATACCAAT
```

```
        710        720        730        740          750
CTAAAGTTAA AAGACAAATA TTTTCAGGCT ATCAATCTGA TATTGATACA
        760        770        780          790        800
CATAATAGAA TTAAGGATGA ATTATGATTA AATTAAAATT TGGTGTTTTT
        810        820        830          840        850
TTTACAGTTT TACTATCTTC AGCATATGCA CATGGAACAC CTCAAAATAT
        860        870        880          890        900
TACTGATTTG TGTGCAGAAT ACCACAACAC ACAAATATAT ACGCTAAATG
        910        920        930          940        950
ATAAGATATT TTCGTATACA GAATCTCTAG CTGGAAAAAG AGAGATGGCT
        960        970        980          990        1000
ATCATTACTT TTAAGAATGG TGCAATTTTT CAAGTAGAAG TACCAAGTAG
        1010       1020       1030       1040          1050
TCAACATATA GATTCACAAA AAAAGCGAT TGAAAGGATG AAGGATACCC
        1060       1070       1080       1090          1100
TGAGGATTGC ATATCTTACT GAAGCTAAAG TCGAAAAGTT ATGTGTATGG
        1110       1120       1130       1140          1150
AATAATAAAA CGCCTCATGC GATTGCCGCA ATTAGTATGG CAAATTAA
```

ainsi que les fragments et dérivés de cette formule, lesdits fragments et dérivés codant pour la totalité ou une partie des sous-unités A et B de la toxine cholérique.

3. Molécule d'ADN recombinante comprenant une séquence d'ADN selon la revendication 1 ou 2.

4. Molécule d'ADN recombinante selon la revendication 3, dans laquelle ladite séquence d'ADN codant pour ladite totalité ou partie des sous-unités A et B de la toxine cholérique est liée de façon opératoire à une séquence de régulation d'expression.

5. Molécule d'ADN recombinante pRIT18014.

6. Hôte transformé par au moins une molécule d'ADN recombinante selon la revendication 4.

7. Souche de E. coli ATCC 39052.

8. Séquence d'ADN selon la revendication 1, comprenant au moins une portion codant pour la totalité ou une partie de la sous-unité A de la toxine cholérique.

9. Fragment de séquence d'ADN selon la revendication 2, choisi dans le groupe comprenant en substance les séquences d'ADN de formule

```
              10         20         30         40         50
ATGGTAAAGA TAATATTTGT GTTTTTTATT TTCTTATCAT CATTTTCATA
              60         70         80         90        100
TGCAAATGAT GATAAGTTAT ATCGGGCAGA TTCTAGACCT CCTGATGAAA
             110        120        130        140        150
TAAAGCAGTC AGGTGGTCTT ATGCCAAGAG GACAGAGTGA GTACTTTGAC
             160        170        180        190        200
CGAGGTACTC AAATGAATAT CAACCTTTAT GATCATGCAA GAGGAACTCA
             210        220        230        240        250
GACGGGATTT GTTAGGCACG ATGATGGATA TGTTTCCACC TCAATTAGTT
             260        270        280        290        300
TGAGAAGTGC CCACTTAGTG GGTCAAACTA TATTGTCTGG TCATTCTACT
             310        320        330        340        350
TATTATATAT ATGTTATAGC CACTGCACCC AACATGTTTA ACGTTAATGA
             360        370        380        390        400
TGTATTAGGG GCATACAGTC CTCATCCAGA TGAACAAGAA GTTTCTGCTT
             410        420        430        440        450
TAGGTGGGAT TCCATACTCC CAAATATATG GATGGTATCG AGTTCATTTT
             460        470        480        490        500
GGGGTGCTTG ATGAACAATT ACATCGTAAT AGGGGCTACA GAGATAGATA
             510        520        530        540        550
TTACAGTAAC TTAGATATTG CTCCAGCAGC AGATGGTTAT GGATTGGCAG
             560        570        580        590        600
GTTTCCCTCC GGAGCATAGA GCTTGGAGGG AAGAGCCGTG GATTCATCAT
             610        620        630        640        650
GCACCGCCGG GTTGTGGGAA TGCTCCAAGA TCATCGATGA GTAATACTTG
             660        670        680        690        700
CGATGAAAAA ACCCAAAGTC TAGGTGTAAA ATTCCTTGAC GAATACCAAT
             710        720        730        740        750
CTAAAGTTAA AAGACAAATA TTTTCAGGCT ATCAATCTGA TATTGATACA
```

```
              760       770       780       790       800
CATAATAGAA TTAAGGATGA ATTATGA
```

ainsi que les fragments et dérivés de cette formule, lesdits fragments et dérivés codant pour la totalité ou une partie de la sous-unité A de la toxine cholérique.

10. Molécule d'ADN recombinante comprenant une séquence d'ADN selon la revendication 8 ou 9.

11. Molécule d'ADN recombinante selon la revendication 10, dans laquelle ladite séquence d'ADN codant pour ladite totalité ou partie de la sous-unité A de la toxine cholérique est liée de façon opératoire à une séquence de régulation d'expression.

12. Molécule d'ADN recombinante pRIT10841.

13. Hôte transformé par au moins une molécule d'ADN recombinante selon la revendication 11.

14. Souche de E. coli ATCC 39053.

15. Séquence d'ADN selon la revendication 1 comprenant au moins une portion codant pour la totalité ou une partie de la sous-unité B de la toxine cholérique.

16. Fragment de séquence d'ADN selon la revendication 2, choisi dans le groupe comprenant en substance les séquences d'ADN de formule

```
                             780       790       800
                         ATGATTA AATTAAAATT TGGTGTTTTT
        810       820       830       840       850
   TTTACAGTTT TACTATCTTC AGCATATGCA CATGGAACAC CTCAAAATAT
        860       870       880       890       900
   TACTGATTTG TGTGCAGAAT ACCACAACAC ACAAATATAT ACGCTAAATG
        910       920       930       940       950
   ATAAGATATT TTCGTATACA GAATCTCTAG CTGGAAAAAG AGAGATGGCT
        960       970       980       990       1000
   ATCATTACTT TTAAGAATGG TGCAATTTTT CAAGTAGAAG TACCAAGTAG
        1010      1020      1030      1040      1050
   TCAACATATA GATTCACAAA AAAAAGCGAT TGAAAGGATG AAGGATACCC
```

```
        1060      1070      1080      1090      1100
TGAGGATTGC ATATCTTACT GAAGCTAAAG TCGAAAAGTT ATGTGTATGG
        1110      1120      1130      1140      1150
AATAATAAAA CGCCTCATGC GATTGCCGCA ATTAGTATGG CAAATTAA
```

ainsi que les fragments et dérivés de cette formule, lesdits fragments et dérivés codant pour la totalité ou une partie de la sous-unité B de la toxine cholérique.

17. Molécule d'ADN recombinante comprenant une séquence d'ADN selon la revendication 15 ou 16.

18. Molécule d'ADN recombinante selon la revendication 17, dans laquelle ladite séquence d'ADN codant pour ladite totalité ou partie de la sous-unité B de la toxine cholérique est liée de façon opératoire à une séquence de régulation d'expression.

19. Molécule d'ADN recombinante pRIT10810.

20. Hôte transformé par au moins une molécule d'ADN recombinante selon la revendication 18.

21. Souche de E. coli ATCC 39051.

22. Composition destinée à stimuler la protection contre l'infection à V. cholerae chez les humains comprenant au moins une quantité efficace d'une anatoxine de la sous-unité A de la toxine cholérique produite par un hôte selon la revendication 13.

23. Composition suivant la revendication 22, dans laquelle la sous-unité A de la toxine cholérique est produite par la souche de E. coli ATCC 39053.

24. Composition destinée à stimuler la protection contre l'infection à V. cholerae chez les humains comprenant au moins une quantité efficace d'une anatoxine de la sous-unité B de la toxine cholérique produite par un hôte selon la revendication 20.

25. Composition destinée à stimuler la protection contre l'infection à V. cholerae chez les humains comprenant au moins une quantité efficace de la sous-unité B de la toxine cholérique produite par un hôte selon la revendication 20.

26. Composition selon la revendication 24 ou 25, dans laquelle la sous-unité B de la toxine cholérique est produite par la souche de E. coli ATCC 39051.

27. Composition destinée à stimuler la protection contre l'infection à V. cholerae chez les humains comprenant au moins une quantité efficace d'une anatoxine des sous-unités A et B de la toxine cholérique produite par un hôte selon la revendication 6.

28. Composition selon la revendication 27, dans laquelle les sous-unités A et B de la toxine cholérique sont produites par la souche d'E. coli ATCC 39052.

29. Procédé de préparation d'une molécule d'ADN recombinante selon la revendication 10, 11 ou 12 caractérisé en ce qu'on met à digérer l'ADN d'une souche de V. cholerae produisant la sous-unité A de la toxine cholérique par l'endonucléase ClaI, on isole un fragment ayant une hybridation spécifique à l'égard d'une sonde eltA, on mélange ledit fragment avec un vecteur possédant un site pour l'endonucléase de restriction ClaI et préalablement digéré par ClaI et on insère le fragment.

30. Procédé de préparation de la sous-unité A de la toxine cholérique caractérisé en ce que on transforme un hôte approprié à l'aide d'une molécule d'ADN recombinante selon la revendication 3, 4 ou 5, on cultive l'hôte transformé et on recueille ladite sous-unité A de la toxine cholérique.

31. Procédé selon la revendication 30 caractérisé en ce que on insère la molécule d'ADN recombinante à un site pour l'endonucléase de restriction ClaI d'un vecteur approprié.

32. Procédé selon la revendication 31, dans lequel le vecteur approprié est le plasmide pBR322.

33. Procédé selon la revendication 30, dans lequel l'hôte approprié est une souche de E. coli K12 ou une souche non toxigénique de V. cholerae.

34. Procédé de préparation d'une molécule d'ADN recombinante selon la revendication 17, 18 ou 19, caractérisée en ce que on met à digérer l'ADN d'une souche de V. cholerae produisant la sous-unité B de la toxine cholérique par l'endonucléase ClaI, on isole un fragment montrant une hybridation spécifique à l'égard d'une sonde eltB, on mélange ledit fragment avec un vecteur possédant un site pour l'endonucléase de restriction ClaI et préalablement digéré par ClaI et on insère le fragment.

35. Procédé de préparation de la sous-unité B de la toxine cholérique caractérisé en ce que on transforme un hôte approprié à l'aide d'une molécule d'ADN recombinante selon la revendication 17, 18 ou 19, on cultive l'hôte transformé et on recueille ladite sous-unité B de la toxine cholérique.

36. Procédé selon la revendication 35 caractérisé en ce que on insère la molécule d'ADN recombinante à un site pour l'endonucléase de restriction ClaI d'un vecteur approprié.

37. Procédé selon la revendication 35, dans lequel le vecteur approprié est le plasmide pBR322.

38. Procédé selon la revendication 35, dans lequel l'hôte approprié est une souche d'E. coli K12 ou une souche non toxigénique de V. cholerae.

39. Procédé de préparation d'une molécule d'ADN recombinante selon la revendication 3, 4 ou 5, caractérisé en ce que on met à digérer une séquence d'ADN qui encode la sous-unité B de la toxine cholérique, séquentiellement par l'endonucléase ClaI et par une endonucléase qui ne coupe pas la séquence codant pour la sous-unité B, on isole le fragment résultant, on insère ledit fragment dans un vecteur adéquat, lequel a été digéré séquentiellement par l'endonucléase ClaI et une seconde endonucléase appropriée pour donner une molécule d'ADN recombinante qui est, par la suite, soit digérée par l'endonucléase ClaI, soit digérée séquentiellement par l'endonucléase ClaI et une seconde endonucléase et recombinée avec un fragment obtenu soit par une digestion avec l'endonucléase ClaI ou une digestion séquentielle par l'endonucléase ClaI et une seconde endonucléase à partir de la souche de V. cholerae qui produit la sous-unité A de la toxine cholérique.

40. Procédé de production des sous-unités A et B de la toxine cholérique caractérisé en ce que on transforme un hôte approprié à l'aide d'une molécule d'ADN recombinante selon la revendication 3, 4 ou 5, on cultive l'hôte transformé et on recueille lesdites sous-unités A et B de la toxine cholérique.

41. Procédé selon la revendication 40 caractérisé en ce que on insère un fragment contenant la séquence du gène de structure de la sous-unité A et une séquence de régulation d'expression pour ledit recombinant à un site pour l'endonucléase de restriction ClaI d'un vecteur approprié.

42. Procédé selon la revendication 41, dans lequel le vecteur approprié est le plasmide pRIT10812.

43. Procédé selon la revendication 40, dans lequel l'hôte est une souche de E. coli K12 ou une souche non toxigénique de V. cholerae.

44. Procédé de détection d'une séquence d'ADN codant pour la sous-unité A de la toxine cholérique à partir d'un groupe de séquences d'ADN caractérisé en ce que on sélectionne les séquences d'ADN pour déterminer celles qui s'hybrident à la séquence d'ADN selon la revendication 8 ou 9.

45. Procédé de détection d'une séquence d'ADN codant pour la sous-unité B de la toxine cholérique à partir d'un groupe de séquences d'ADN caractérisé en ce que on sélectionne les séquences d'ADN pour déterminer celles qui s'hybrident à la séquence d'ADN selon la revendication 15 ou 16.

46. Procédé de détection d'une séquence d'ADN codant pour les sous-unités A et B de la toxine cholérique à partir d'un groupe de séquences d'ADN caractérisé en ce que on sélectionne les séquences d'ADN pour déterminer celles qui s'hybrident à la séquence d'ADN selon la revendication 1 ou 2.

REVENDICATIONS POUR L'ETAT CONTRACTANT : AT.

1 Procédé de préparation d'une molécule d'ADN recombinante comprenant une séquence d'ADN qui contient au moins une portion codant pour la totalité ou une partie de la sous-unité A de la toxine cholérique caractérisé en ce que on met à digérer de l'ADN d'une souche de V. cholerae productrice de sous-unité A de la toxine cholérique avec de l'endonucléase ClaI, on isole un fragment qui présente une hybridation spécifique à l'égard d'une sonde eltA, on mélange ledit fragment avec un vecteur possédant un site pour l'endonucléase de restriction ClaI et préalablement digéré par ClaI et on insère le fragment.

2. Procédé selon la revendication 1 dans lequel ladite séquence d'ADN codant pour la totalité ou une partie de la sous-unité A de la toxine cholérique est liée de façon opératoire à une séquence de régulation d'expression.

3. Procédé selon la revendication 1 ou 2 dans lequel la molécule d'ADN recombinante est le pRIT10841.

4. Procédé de préparation de la sous-unité A de la toxine cholérique dans lequel on transforme un hôte approprié à l'aide d'une molécule d'ADN recombinante comprenant au moins une portion codant pour l'entièreté ou une partie de la sous-unité A de la toxine cholérique, on cultive l'hôte transformé et on recueille ladite sous-unité A de la toxine cholérique.

5. Procédé selon la revendication 4 dans lequel la molécule d'ADN recombinante est liée de façon opératoire à une séquence de régulation d'expression.

6. Procédé selon la revendication 4 ou 5 dans lequel la molécule d'ADN recombinante est le pRIT 10841.

7. Procédé selon la revendication 4, 5 ou 6 dans lequel on insère la molécule d'ADN recombinante à un site pour l'endonucléase de restriction ClaI d'un vecteur approprié.

8. Procédé selon la revendication 7 dans lequel
le vecteur approprié est le plasmide pBR322.

9. Procédé selon la revendication 4, 5, 6, 7 ou 8
dans lequel l'hôte approprié est une souche de E. coli
K12 ou une souche non toxigénique de V. cholerae.

10. Procédé selon la revendication 4 dans lequel
l'hôte transformé est la souche de E. coli ATCC 39053.

11. Procédé de préparation d'une molécule d'ADN
recombinante comprenant une séquence d'ADN qui contient
au moins une portion codant pour la totalité ou une
partie de la sous-unité B de la toxine cholérique,
caractérisé en ce que on met à digérer l'ADN d'une souche de V. cholerae productrice de sous-unité B de la
toxine cholérique avec de l'endonucléase ClaI, on isole
un fragment qui présente une hybridation spécifique à
l'égard d'une sonde eltB, on mélange ledit fragment
avec un vecteur possédant un site pour l'endonucléase
de restriction ClaI et préalablement digéré par ClaI et
on insère le fragment.

12. Procédé selon la revendication 11 dans lequel
ladite séquence d'ADN codant pour la totalité ou une
partie de la sous-unité B de la toxine cholérique est
liée de façon opératoire à une séquence de régulation
d'expression.

13. Procédé selon la revendication 11 ou 12 dans
lequel la molécule d'ADN recombinante est le pRIT10810.

14. Procédé de préparation de la sous-unité B de
la toxine cholérique dans lequel on transforme un hôte
approprié à l'aide d'une molécule d'ADN recombinante
comprenant au moins une portion codant pour l'entièreté
ou une partie de la sous-unité B de la toxine cholérique, on cultive l'hôte transformé et on recueille
ladite sous-unité B de la toxine cholérique.

15. Procédé selon la revendication 14 dans lequel la molécule d'ADN recombinante est liée de façon opératoire à une séquence de régulation d'expression.

16. Procédé selon la revendication 14 ou 15, dans lequel la molécule d'ADN recombinante est le pRIT10810.

17. Procédé selon la revendication 15, 15 ou 16 dans lequel on insère la molécule d'ADN recombinante à un site pour l'endonucléase de restriction ClaI d'un vecteur approprié.

18. Procédé selon la revendication 17 dans lequel le vecteur approprié est le plasmide pBR322.

19. Procédé selon la revendication 14, 15, 16, 17 ou 18 dans lequel l'hôte approprié est une souche de E. coli K12 ou une souche non toxigénique de V. cholerae.

20. Procédé selon la revendication 14 dans lequel l'hôte transformé est la souche de E. coli ATCC 39051.

21. Procédé de préparation d'une molécule d'ADN recombinante comprenant une séquence d'ADN qui contient au moins une portion codant pour la totalité ou une partie des sous-unités A et B de la toxine cholérique, caractérisé en ce que on met à digérer une séquence d'ADN codant pour la sous-unité B de la toxine cholérique successivement avec de l'endonucléase ClaI et une endonucléase qui ne coupe pas la séquence codant pour la sous-unité B, on isole le fragment résultant, on insère ledit fragment dans un vecteur adéquat qui a été digéré successivement avec de l'endonucléase ClaI et une seconde endonucléase appropriée pour donner une molécule d'ADN recombinante qui est ensuite digérée soit avec de l'endonucléase ClaI soit successivement avec de l'endonucléase ClaI et une seconde endonucléase et recombinée avec un fragment obtenu par digestion d'une souche de V. cholerae productrice de sous-unité A de la toxine cholérique soit avec l'endonucléase ClaI

soit successivement avec l'endonucléase ClaI et une seconde endonucléase.

22. Procédé selon la revendication 21 dans lequel ladite séquence d'ADN codant pour l'entièreté ou une partie des sous-unités B et A de la toxine cholérique est liée de façon opératoire à une séquence de régulation d'expression.

23. Procédé selon la revendication 21 ou 22 dans lequel la molécule recombinante est le pRIT10814.

24. Procédé de préparation des sous-unités A et B de la toxine cholérique dans lequel on transforme un hôte approprié à l'aide d'une molécule d'ADN recombinante obtenue par le procédé de la revendication 21, 22 ou 23, on cultive l'hôte transformé et on recueille lesdites sous-unités A et B de la toxine cholérique.

25. Procédé selon la revendication 24 dans lequel on insère un fragment contenant la séquence du gène de structure de la sous-unité A et une séquence de régulation d'expression pour ledit recombinant à un site pour l'endonucléase de restriction ClaI d'un vecteur approprié.

26. Procédé selon la revendication 24 ou 25 dans lequel le vecteur approprié est le plasmide pRIT 10812.

27. Procédé selon la revendication 21, 22 ou 23 dans lequel l'hôte est une souche de E. coli K12 ou une souche non toxigénique de V. cholerae.

28. Procédé de détection d'une séquence d'ADN codant pour la sous-unité A de la toxine cholérique à partir d'un groupe de séquences d'ADN, caractérisé en ce que on sélectionne les séquences d'ADN pour déterminer celles qui s'hybrident à la séquence d'ADN obtenue par le procédé de la revendication 1.

29. Procédé de détection d'une séquence d'ADN codant pour la sous-unité B de la toxine cholérique à partir d'un groupe de séquences d'ADN, caractérisé en ce que on sélectionne les séquences d'ADN pour déterminer celles qui s'hybrident à la séquence d'ADN obtenue
par le procédé de la revendication 11.

30. Procédé de détection d'une séquence d'ADN
codant pour les sous-unités A et B de la toxine cholérique à partir d'un groupe de séquences d'ADN, caractérisé en ce que on sélectionne les séquences d'ADN pour
déterminer celles qui s'hybrident à la séquence d'ADN
obtenue par le procédé de la revendication 21.

FIG 1.

FIG 2.

FIG 3.

FIG 4.

0095452

FIG 5.

```
        10         20         30         40         50
ATGGTAAAGA TAATATTTGT GTTTTTTATT TTCTTATCAT CATTTTCATA
        60         70         80         90        100
TGCAAATGAT GATAAGTTAT ATCGGGCAGA TTCTAGACCT CCTGATGAAA
       110        120        130        140        150
TAAAGCAGTC AGGTGGTCTT ATGCCAAGAG GACAGAGTGA GTACTTTGAC
       160        170        180        190        200
CGAGGTACTC AAATGAATAT CAACCTTTAT GATCATGCAA GAGGAACTCA
       210        220        230        240        250
GACGGGATTT GTTAGGCACG ATGATGGATA TGTTTCCACC TCAATTAGTT
       260        270        280        290        300
TGAGAAGTGC CCACTTAGTG GGTCAAACTA TATTGTCTGG TCATTCTACT
       310        320        330        340        350
TATTATATAT ATGTTATAGC CACTGCACCC AACATGTTTA ACGTTAATGA
       360        370        380        390        400
TGTATTAGGG GCATACAGTC CTCATCCAGA TGAACAAGAA GTTTCTGCTT
       410        420        430        440        450
TAGGTGGGAT TCCATACTCC CAAATATATG GATGGTATCG AGTTCATTTT
       460        470        480        490        500
GGGGTGCTTG ATGAACAATT ACATCGTAAT AGGGGCTACA GAGATAGATA
       510        520        530        540        550
TTACAGTAAC TTAGATATTG CTCCAGCAGC AGATGGTTAT GGATTGGCAG
       560        570        580        590        600
GTTTCCCTCC GGAGCATAGA GCTTGGAGGG AAGAGCCGTG GATTCATCAT
       610        620        630        640        650
GCACCGCCGG GTTGTGGGAA TGCTCCAAGA TCATCGATGA GTAATACTTG
       660        670        680        690        700
CGATGAAAAA ACCCAAAGTC TAGGTGTAAA ATTCCTTGAC GAATACCAAT
       710        720        730        740        750
CTAAAGTTAA AAGACAAATA TTTTCAGGCT ATCAATCTGA TATTGATACA
       760        770        780        790        800
CATAATAGAA TTAAGGATGA ATTATGATTA AATTAAAATT TGGTGTTTTT
       810        820        830        840        840
TTTACAGTTT TACTATCTTC AGCATATGCA CATGGAACAC CTCAAAATAT
       860        870        880        890        900
TACTGATTTG TGTGCAGAAT ACCACAACAC ACAAATATAT ACGCTAAATG
       910        920        930        940        950
ATAAGATATT TTCGTATACA GAATCTCTAG CTGGAAAAAG AGAGATGGCT
       960        970        980        990       1000
ATCATTACTT TTAAGAATGG TGCAATTTTT CAAGTAGAAG TACCAAGTAG
      1010       1020       1030       1040       1050
TCAACATATA GATTCACAAA AAAAAGCGAT TGAAAGGATG AAGGATACCC
      1060       1070       1080       1090       1100
TGAGGATTGC ATATCTTACT GAAGCTAAAG TCGAAAAGTT ATGTGTATGG
      1110       1120       1130       1140       1150
AATAATAAAA CGCCTCATGC GATTGCCGCA ATTAGTATGG CAAATTAA
```

FIG 6.